# EUROPEAN PATENT APPLICATION

(11) **EP 4 549 560 A1**
(43) Date of publication of application: **07.05.2025**
(21) Application number: 24799830.5
(22) Date of filing: 22.02.2024
(51) Int. Cl.: C12N 9/18, C12N 15/55, C12N 15/70, C12N 15/64, C12N 1/21, B09B 3/60, C12R 1/19, B09B 101/75

(54) **ISPETASE-8×CHIMERA RECOMBINASE, AND ENCODING GENE, RECOMBINANT PLASMID ENGINEERING BACTERIUM AND USE THEREOF**

(30) Priority: 28.09.2023 CN 202311275023
(71) Applicant: Beijing Meihao Biotechnology Co., Ltd., Beijing 102600 (CN)
(72) Inventor: QI, Wei, Beijing 102600 (CN); YOU, Shengping, Beijing 102600 (CN); WANG, Mengfan, Beijing 102600 (CN); ZHANG, Jiaxing, Beijing 102600 (CN); SHI, Wenhui, Beijing 102600 (CN); YIN, Qingdian, Beijing 102600 (CN); SU, Rongxin, Beijing 102600 (CN)
(74) Representative: Isarpatent
(86) International application number: PCT/CN2024/078000
(87) International publication number: WO 2024/227372

(57) **Abstract**

An IsPETase-8×Chimera recombinase includes an amino acid sequence modified from an amino acid sequence of a wild-type PET hydrolase by replacing at least one of the amino acid sequences set forth in SEQ ID NOs: 1 to 8; the amino acid sequence of the wild-type PET hydrolase includes the amino acid sequences set forth in SEQ ID NOs: 1 to 8. Compared with the native PET enzyme, the recombinase has a relatively high activity and thermal stability, which is of great significance for achieving efficient degradation of PET. This application also provides an encoding gene which encodes the recombinase, as well as a recombinant plasmid containing the encoding gene and an engineered strain containing the recombinant plasmid.

## Description

### TECHNICAL FIELD

The application relates to the technical field of enzyme engineering, in particular to an IsPETase-8×Chimera recombinase, an encoding gene, a recombinant plasmid, an engineered strain and applications.

### BACKGROUND

As the amount of plastic consumption increases, the impact of plastic waste on the environment has attracted widespread global attention. Polyethylene terephthalate, PET, is one of the most demanded polyester plastics. Generally, major methods for processing PET include landfilling, incineration, pyrolysis, and chemical degradation, all of which consume a large amount of energy and may cause secondary pollution. However, PET is formed by ester bonds of terephthalic acid, TPA, and ethylene glycol, EG, so it can be decomposed by hydrolysis of various enzymes, such as lipases and cutinases. If PET is degraded and TPA is recovered, not only can the impact of PET on the environment be reduced, but the recovered TPA can also bring additional social and economic benefits. Thus, complete depolymerization of PET into monomers is a necessary step to reduce environmental pollution caused by PET, and to obtain a high value-added product TPA.

The biological treatment technology is gradually applied to the treatment of waste plastics due to its characteristics such as high efficiency of degradation, low cost and environmental friendliness. Over the past few years, enzyme-based technology has made significant advances in the treatment of PET waste, providing an environmentally friendly and cost-effective new choice. A variety of enzymes capable of hydrolyzing PET have been discovered, such as esterases, lipases, and cutinases, etc. However, the activity of these PET hydrolases at room temperature is very low, so it is not possible to degrade the remaining PET plastic in situ in natural environment. PETase from bacterial Ideonella sakaiensis, also known as IsPETase, is an enzyme for degrading PET. IsPETase can effectively hydrolyze PET into TPA and other products at room temperature. However, thermal stability and activity at high temperature of IsPETase are poor, and the practical application thereof is still limited.

### SUMMARY

### TECHNICAL PROBLEMS

The present application provides an IsPETase chimera (IsPETase-8×Chimera recombinase), an encoding gene, a recombinant plasmid, an engineered strain and applications. The recombinase has a relatively high activity and thermal stability, which remedies the deficiency of the existing natural PET hydrolases.

### TECHNICAL SOLUTIONS

In order to achieve the object, the technical solution adopted by the present application is as follows:
In a first aspect, the present application provides an IsPETase-8×Chimera recombinase, and the IsPETase-8×Chimera recombinase includes an amino acid sequence modified from an amino acid sequence of a wild-type PET hydrolase (set forth in SEQ ID NO: 21) by replacing at least one of the amino acid sequences set forth in SEQ ID NOs: 1 to 8; where
the amino acid sequence set forth in SEQ ID NO: 1 is replaced by the amino acid sequence set forth in SEQ ID NO: 9 or SEQ ID NO: 10;
the amino acid sequence set forth in SEQ ID NO: 2 is replaced by the amino acid sequence set forth in SEQ ID NO: 11 or SEQ ID NO: 12;
the amino acid sequence set forth in SEQ ID NO: 3 is replaced by the amino acid sequence set forth in SEQ ID NO: 13 or SEQ ID NO: 14;
the amino acid sequence set forth in SEQ ID NO: 4 is replaced by the amino acid sequence set forth in SEQ ID NO: 15;
the amino acid sequence set forth in SEQ ID NO: 5 is replaced by the amino acid sequence set forth in SEQ ID NO: 16 or SEQ ID NO: 17;
the amino acid sequence set forth in SEQ ID NO: 6 is replaced by the amino acid sequence set forth in SEQ ID NO: 18;
the amino acid sequence set forth in SEQ ID NO: 7 is replaced by the amino acid sequence set forth in SEQ ID NO: 19; and
the amino acid sequence set forth in SEQ ID NO: 8 is replaced by the amino acid sequence set forth in SEQ ID NO: 20.

It should be noted that the amino acid sequences set forth in SEQ ID No. 1 to 8 are partial sequences of IsPETase (the amino acid sequence is set forth in SEQ ID No. 21). A peptide segment consisting of the first 26 amino acid residues in the sequence of IsPETase is a signal peptide, and in order to prevent the signal peptide from affecting the correct folding of the protein, it needs to be removed in advance. Thus, the signal peptide is not included in the IsPETase-8 × Chimera recombinase.

The IsPETase-8×Chimera recombinase constructed in the present application has a high activity and thermal stability, which makes up for the limitations that the natural IsPETase enzyme has a poor thermal stability and a low reaction activity in practical applications. It can be applied to PET hydrolysis catalytic reactions that have important value in the fields of environment and resources.

Based on the first aspect, in some embodiments, the amino acid sequences set forth in SEQ ID NOs: 2, 3, 4, 5 and 7 are replaced.

Based on the first aspect, as a more preferred technical solution, the amino acid sequence set forth in SEQ ID NO: 3 is replaced by the amino acid sequence set forth in SEQ ID NO: 14.

As an example, the amino acid sequence set forth in SEQ ID NO: 1 is replaced by the amino acid sequence set forth in SEQ ID NO: 9, the amino acid sequence set forth in SEQ ID NO: 2 is replaced by the amino acid sequence set forth in SEQ ID NO: 12, the amino acid sequence set forth in SEQ ID NO: 3 is replaced by the amino acid sequence set forth in SEQ ID NO: 14, the amino acid sequence set forth in SEQ ID NO: 4 is replaced by the amino acid sequence set forth in SEQ ID NO: 15, the amino acid sequence set forth in SEQ ID NO: 5 is replaced by the amino acid sequence set forth in SEQ ID NO: 17, the amino acid sequence set forth in SEQ ID NO: 6 is replaced by the amino acid sequence set forth in SEQ ID NO: 18, the amino acid sequence set forth in SEQ ID NO: 7 is replaced by the amino acid sequence set forth in SEQ ID NO: 19, and the amino acid sequence set forth in SEQ ID NO: 8 is replaced by the amino acid sequence set forth in SEQ ID NO: 20.

As an example, the amino acid sequence set forth in SEQ ID NO: 1 is replaced by the amino acid sequence set forth in SEQ ID NO: 10, the amino acid sequence set forth in SEQ ID NO: 2 is replaced by the amino acid sequence set forth in SEQ ID NO: 12, the amino acid sequence set forth in SEQ ID NO: 3 is replaced by the amino acid sequence set forth in SEQ ID NO: 14, the amino acid sequence set forth in SEQ ID NO: 4 is replaced by the amino acid sequence set forth in SEQ ID NO: 15, the amino acid sequence set forth in SEQ ID NO: 5 is replaced by the amino acid sequence set forth in SEQ ID NO: 17, the amino acid sequence set forth in SEQ ID NO: 6 is replaced by the amino acid sequence set forth in SEQ ID NO: 18, the amino acid sequence set forth in SEQ ID NO: 7 is replaced by the amino acid sequence set forth in SEQ ID NO: 19, and the amino acid sequence set forth in SEQ ID NO: 8 is replaced by the amino acid sequence set forth in SEQ ID NO: 20.

As an example, the amino acid sequence set forth in SEQ ID NO: 1 is replaced by the amino acid sequence set forth in SEQ ID NO: 9, the amino acid sequence set forth in SEQ ID NO: 2 is replaced by the amino acid sequence set forth in SEQ ID NO: 12, the amino acid sequence set forth in SEQ ID NO: 3 is replaced by the amino acid sequence set forth in SEQ ID NO: 14, the amino acid sequence set forth in SEQ ID NO: 4 is replaced by the amino acid sequence set forth in SEQ ID NO: 15, the amino acid sequence set forth in SEQ ID NO: 5 is replaced by the amino acid sequence set forth in SEQ ID NO: 16, the amino acid sequence set forth in SEQ ID NO: 7 is replaced by the amino acid sequence set forth in SEQ ID NO: 19, and the amino acid sequence set forth in SEQ ID NO: 8 is replaced by the amino acid sequence set forth in SEQ ID NO: 20.

As an example, the amino acid sequence set forth in SEQ ID NO: 2 is replaced by the amino acid sequence set forth in SEQ ID NO: 12, the amino acid sequence set forth in SEQ ID NO: 3 is replaced by the amino acid sequence set forth in SEQ ID NO: 14, the amino acid sequence set forth in SEQ ID NO: 4 is replaced by the amino acid sequence set forth in SEQ ID NO: 15, the amino acid sequence set forth in SEQ ID NO: 5 is replaced by the amino acid sequence set forth in SEQ ID NO: 16, the amino acid sequence set forth in SEQ ID NO: 6 is replaced by the amino acid sequence set forth in SEQ ID NO: 18, the amino acid sequence set forth in SEQ ID NO: 7 is replaced by the amino acid sequence set forth in SEQ ID NO: 19, and the amino acid sequence set forth in SEQ ID NO: 8 is replaced by the amino acid sequence set forth in SEQ ID NO: 20.

As an example, the amino acid sequence set forth in SEQ ID NO: 1 is replaced by the amino acid sequence set forth in SEQ ID NO: 9, the amino acid sequence set forth in SEQ ID NO: 2 is replaced by the amino acid sequence set forth in SEQ ID NO: 11, the amino acid sequence set forth in SEQ ID NO: 3 is replaced by the amino acid sequence set forth in SEQ ID NO: 14, the amino acid sequence set forth in SEQ ID NO: 4 is replaced by the amino acid sequence set forth in SEQ ID NO: 15, the amino acid sequence set forth in SEQ ID NO: 5 is replaced by the amino acid sequence set forth in SEQ ID NO: 16, the amino acid sequence set forth in SEQ ID NO: 6 is replaced by the amino acid sequence set forth in SEQ ID NO: 18, the amino acid sequence set forth in SEQ ID NO: 7 is replaced by the amino acid sequence set forth in SEQ ID NO: 19, and the amino acid sequence set forth in SEQ ID NO: 8 is replaced by the amino acid sequence set forth in SEQ ID NO: 20.

In a second aspect, the present application also provides an application of the IsPETase-8×Chimera recombinase for hydrolyzing PET. The application includes: adding PET to a glycine-sodium hydroxide, NaOH, buffer with a pH of 8.0 to 10.0 and a concentration of 40 millimoles per liter, mM, to 60 mM so that a final concentration of PET is 60 milligrams per milliliter, mg/mL, to 100 mg/mL; adding the IsPETase-8×Chimera recombinase to the solution so that a final concentration of the IsPETase-8×Chimera recombinase is 400 nanomoles per liter, nM, to 600 nM; and incubating the mixture for 1 to 3 days at a temperature of 30°C to 50°C and a rotation speed of 100 revolutions per minute, rpm, to 300 rpm.

In a third aspect, the present application also provides an encoding gene (a nucleic acid) which encodes the IsPETase-8×Chimera recombinase.

In a fourth aspect, the present application also provides a recombinant plasmid which includes the nucleic acid.

In a fifth aspect, the present application also provides a method for constructing the recombinant plasmid, and the method includes:
using plasmid pET-22b-IsPETase, plasmid pET-22b-TfCut2 and plasmid pET-22b-LCC as templates, and performing PCR to obtain linear vector fragments corresponding to each template; mixing each of the linear vector fragments corresponding to each template with a ligase to form circular recombinant plasmids; transforming each of the circular recombinant plasmids into a T1 competent cell, and obtaining a recombinant plasmid pET-22b-IsPETase-cSP, a recombinant plasmid pET-22b-TfCut2-cSP and a recombinant plasmid pET-22b-LCC-cSP by ampicillin-containing medium screening and plasmid extraction; a nucleotide sequence of an IsPETase-cSP gene in the recombinant plasmid pET-22b-IsPETase-cSP is set forth in SEQ ID NO: 51, and an amino acid sequence of an IsPETase-cSP enzyme is set forth in SEQ ID NO: 52; a nucleotide sequence of a TfCut2-cSP gene in the recombinant plasmid pET-22b-TfCut2-cSP is set forth in SEQ ID NO: 53, and an amino acid sequence of a TfCut2-cSP enzyme is set forth in SEQ ID NO: 54; a nucleotide sequence of a LCC-cSP gene in the recombinant plasmid pET-22b-LCC-cSP is set forth in SEQ ID NO: 55, and an amino acid sequence of a LCC-cSP enzyme is set forth in SEQ ID NO: 56. And
Using at least two of the recombinant plasmid pET-22b-IsPETase-cSP, the recombinant plasmid pET-22b-TfCut2-cSP and the recombinant plasmid pET-22b-LCC-cSP as templates to perform PCR separately to generate PCR amplified fragments, ligating the PCR amplified fragments to generate a ligation product, transforming the ligation product into a T1 competent cell, screening using the ampicillin-containing medium and performing plasmid extraction to obtain the recombinant plasmid.

In a sixth aspect, the present application also provides an engineered strain which includes the recombinant plasmid.

In a seventh aspect, the present application also provides a method for constructing an engineered strain, and the method includes: transforming the recombinant plasmid into a BL21(DE3) competent cell, and culturing the BL21(DE3) competent cell using an ampicillin-containing medium to obtain a positive recombinant.

In an eighth aspect, also encompassed by the present application is a use of the engineered strain for hydrolyzing PET. The use includes: culturing the engineered strain in an LB liquid medium to reach OD600 ranging from 0.7 to 0.9, adding 0.05% to 0.2% of IPTG (isopropylthio-β-galactoside) to the medium, cooling to 14°C to 20°C to induce expression for 16 hours, h, to 24 h, centrifuging, and collecting wet cells of the engineered strain;
after breaking down the wet cells of the engineered strain, centrifuging to remove cell debris, eluting the centrifuged supernatant with an elution buffer as an elution solvent by using a Ni-NTA packing column, collecting an eluate, and concentrating the eluate to obtain a protein concentrate containing proteins;
adding the protein concentrate to a glycine-sodium hydroxide buffer with a pH of 8.0 to 10.0 and a concentration of 40 mM to 60 mM, so that the concentration of the proteins is 400 nM to 600 nM, and hydrolyzing PET at 30°C to 60°C; wherein a concentration of glycine in the glycine-sodium hydroxide buffer is 50 mM to 200 mM.

### ADVANTAGEOUS EFFECTS OF THE APPLICATION

The IsPETase-8×Chimera recombinase provided in the present application has a relatively high activity and thermal stability. The maximum melting temperature, Tm, can be increased up to 82.71°C which is 36°C higher than the Tm of natural IsPETase. The TPA production can reach up to 4856.49 micromoles per liter, µM, which is far higher than that of the wild-type PET hydrolases, such as PET hydrolases IsPETase, PET hydrolases TfCut2 or PET hydrolases LCC at the same temperature. The amino acid fragments of the recombinase respectively come from different native enzyme parents. However, compared with the native enzyme, the recombinase has a new function which the native enzyme does not possess, which is of great significance for achieving efficient degradation of PET. The recombinase can be applied in fields such as plastic waste conversion, resource utilization and environmental protection, and has a wide range of application value.

### DETAILED DESCRIPTION OF ILLUSTRATIVE EMBODIMENTS

In order to better illustrate the present application, further details are given below through specific embodiments and drawings.

The degradation of PET can reduce environmental pollution, and obtain a high value-added product TPA. The enzyme treatment technology can play a role in PET degradation. Currently, a variety of PET hydrolases have been discovered, such as esterases, lipases and cutinases. However, these hydrolases have a low activity at room temperature. IsPETase is capable of effectively hydrolyzing PET at room temperature, but it mainly hydrolyzes amorphous PET. The native IsPETase has a poor stability and activity at high temperature, and its activity will be mostly lost after being incubated at 37°C for 24 hours, which is not sufficient to maintain a highly effective substrate degradation activity at a higher reaction temperature, seriously limiting its application range.

Based on the study of IsPETase, and combined with rational design and directed evolution, the present application provides an IsPETase-8×Chimera recombinase having high activity and thermal stability by replacing different domains of IsPETase with other amino acid sequences to alter its performance. The recombinase is obtained by replacing at least one of amino acid sequences set forth in SEQ ID NOs: 1 to 8 on the basis of IsPETase (amino acid sequence of IsPETase set forth in SEQ ID NO: 21); where
the amino acid sequence set forth in SEQ ID NO: 1 is replaced by the amino acid sequence set forth in SEQ ID NO: 9 or SEQ ID NO: 10;
the amino acid sequence set forth in SEQ ID NO: 2 is replaced by the amino acid sequence set forth in SEQ ID NO: 11 or SEQ ID NO: 12;
the amino acid sequence set forth in SEQ ID NO: 3 is replaced by the amino acid sequence set forth in SEQ ID NO: 13 or SEQ ID NO: 14;
the amino acid sequence set forth in SEQ ID NO: 4 is replaced by the amino acid sequence set forth in SEQ ID NO: 15;
the amino acid sequence set forth in SEQ ID NO: 5 is replaced by the amino acid sequence set forth in SEQ ID NO: 16 or SEQ ID NO: 17;
the amino acid sequence set forth in SEQ ID NO: 6 is replaced by the amino acid sequence set forth in SEQ ID NO: 18;
the amino acid sequence set forth in SEQ ID NO: 7 is replaced by the amino acid sequence set forth in SEQ ID NO: 19; and
the amino acid sequence set forth in SEQ ID NO: 8 is replaced by the amino acid sequence set forth in SEQ ID NO: 20.

The present embodiment also provides a method for constructing the IsPETase-8×Chimera recombinase and an application of the IsPETase-8×Chimera recombinase in the hydrolysis of PET.

The present embodiment also provides a gene (nucleic acid) encoding the IsPETase-8×Chimera recombinase.

The present embodiment also provides a recombinant plasmid which includes the nucleic acid, a method for constructing the recombinant plasmid, and an engineered strain constructed using the recombinant plasmid.

Also provided in the embodiment of the present application is a method for constructing the engineered strain and an application in the hydrolysis of PET.

The solution of the present application is illustrated below with reference to specific embodiments.

The reagents in the following examples, if not specified, are either commercially available or obtained according to methods well-known in the art.

### Example 1.

The example of the present application provides an IsPETase-8×Chimera recombinase with a high activity and thermal stability. The IsPETase-8×Chimera recombinase was obtained on the basis of IsPETase (amino acid sequence of IsPETase set forth in SEQ ID NO: 21) by replacing the amino acid sequence set forth in SEQ ID NO: 1 with the amino acid sequence set forth in SEQ ID NO: 9, replacing the amino acid sequence set forth in SEQ ID NO: 2 with the amino acid sequence set forth in SEQ ID NO: 11, replacing the amino acid sequence set forth in SEQ ID NO: 3 with the amino acid sequence set forth in SEQ ID NO: 13, replacing the amino acid sequence set forth in SEQ ID NO: 4 with the amino acid sequence set forth in SEQ ID NO: 15, replacing the amino acid sequence set forth in SEQ ID NO: 5 with the amino acid sequence set forth in SEQ ID NO: 16, replacing the amino acid sequence set forth in SEQ ID NO: 6 with the amino acid sequence set forth in SEQ ID NO: 18, replacing the amino acid sequence set forth in SEQ ID NO: 7 with the amino acid sequence set forth in SEQ ID NO: 19, and replacing the amino acid sequence set forth in SEQ ID NO: 8 with the amino acid sequence set forth in SEQ ID NO: 20. The nucleotide sequence encoding the IsPETase-8×Chimera recombinase was set forth in SEQ ID NO.22.

### Example 2.

The example of the present application provides an IsPETase-8×Chimera recombinase with a high activity and thermal stability. The IsPETase-8×Chimera recombinase was obtained on the basis of IsPETase (amino acid sequence of IsPETase set forth in SEQ ID NO: 21) by replacing the amino acid sequence set forth in SEQ ID NO: 1 with the amino acid sequence set forth in SEQ ID NO: 9, replacing the amino acid sequence set forth in SEQ ID NO: 2 with the amino acid sequence set forth in SEQ ID NO: 11, replacing the amino acid sequence set forth in SEQ ID NO: 3 with the amino acid sequence set forth in SEQ ID NO: 14, replacing the amino acid sequence set forth in SEQ ID NO: 4 with the amino acid sequence set forth in SEQ ID NO: 15, replacing the amino acid sequence set forth in SEQ ID NO: 5 with the amino acid sequence set forth in SEQ ID NO: 16, replacing the amino acid sequence set forth in SEQ ID NO: 7 with the amino acid sequence set forth in SEQ ID NO: 19, and replacing the amino acid sequence set forth in SEQ ID NO: 8 with the amino acid sequence set forth in SEQ ID NO: 20. The nucleotide sequence encoding the IsPETase-8×Chimera recombinase was set forth in SEQ ID NO.23.

### Example 3.

The example of the present application provides an IsPETase-8×Chimera recombinase with a high activity and thermal stability. The IsPETase-8×Chimera recombinase was obtained on the basis of IsPETase (amino acid sequence of IsPETase set forth in SEQ ID NO: 21) by replacing the amino acid sequence set forth in SEQ ID NO: 1 with the amino acid sequence set forth in SEQ ID NO: 10, replacing the amino acid sequence set forth in SEQ ID NO: 2 with the amino acid sequence set forth in SEQ ID NO: 11, replacing the amino acid sequence set forth in SEQ ID NO: 3 with the amino acid sequence set forth in SEQ ID NO: 14, replacing the amino acid sequence set forth in SEQ ID NO: 4 with the amino acid sequence set forth in SEQ ID NO: 15, replacing the amino acid sequence set forth in SEQ ID NO: 5 with the amino acid sequence set forth in SEQ ID NO: 17, replacing the amino acid sequence set forth in SEQ ID NO: 6 with the amino acid sequence set forth in SEQ ID NO: 18, replacing the amino acid sequence set forth in SEQ ID NO: 7 with the amino acid sequence set forth in SEQ ID NO: 19, and replacing the amino acid sequence set forth in SEQ ID NO: 8 with the amino acid sequence set forth in SEQ ID NO: 20. The nucleotide sequence encoding the IsPETase-8×Chimera recombinase was set forth in SEQ ID NO.24.

### Example 4.

The example of the present application provides an IsPETase-8×Chimera recombinase with a high activity and thermal stability. The IsPETase-8×Chimera recombinase was obtained on the basis of IsPETase (amino acid sequence of IsPETase set forth in SEQ ID NO: 21) by replacing the amino acid sequence set forth in SEQ ID NO: 1 with the amino acid sequence set forth in SEQ ID NO: 9, replacing the amino acid sequence set forth in SEQ ID NO: 2 with the amino acid sequence set forth in SEQ ID NO: 12, replacing the amino acid sequence set forth in SEQ ID NO: 3 with the amino acid sequence set forth in SEQ ID NO: 14, replacing the amino acid sequence set forth in SEQ ID NO: 4 with the amino acid sequence set forth in SEQ ID NO: 15, replacing the amino acid sequence set forth in SEQ ID NO: 5 with the amino acid sequence set forth in SEQ ID NO: 17, replacing the amino acid sequence set forth in SEQ ID NO: 6 with the amino acid sequence set forth in SEQ ID NO: 18, and replacing the amino acid sequence set forth in SEQ ID NO: 7 with the amino acid sequence set forth in SEQ ID NO: 19. The nucleotide sequence encoding the IsPETase-8×Chimera recombinase was set forth in SEQ ID NO.25.

### Example 5.

The example of the present application provides an IsPETase-8×Chimera recombinase with a high activity and thermal stability. The IsPETase-8×Chimera recombinase was obtained on the basis of IsPETase (amino acid sequence of IsPETase set forth in SEQ ID NO: 21) by replacing the amino acid sequence set forth in SEQ ID NO: 1 with the amino acid sequence set forth in SEQ ID NO: 9, replacing the amino acid sequence set forth in SEQ ID NO: 2 with the amino acid sequence set forth in SEQ ID NO: 12, replacing the amino acid sequence set forth in SEQ ID NO: 3 with the amino acid sequence set forth in SEQ ID NO: 13, replacing the amino acid sequence set forth in SEQ ID NO: 4 with the amino acid sequence set forth in SEQ ID NO: 15, replacing the amino acid sequence set forth in SEQ ID NO: 5 with the amino acid sequence set forth in SEQ ID NO: 17, replacing the amino acid sequence set forth in SEQ ID NO: 6 with the amino acid sequence set forth in SEQ ID NO: 18, replacing the amino acid sequence set forth in SEQ ID NO: 7 with the amino acid sequence set forth in SEQ ID NO: 19, and replacing the amino acid sequence set forth in SEQ ID NO: 8 with the amino acid sequence set forth in SEQ ID NO: 20. The nucleotide sequence encoding the IsPETase-8×Chimera recombinase was set forth in SEQ ID NO.26.

### Example 6.

The example of the present application provides an IsPETase-8×Chimera recombinase with a high activity and thermal stability. The IsPETase-8×Chimera recombinase was obtained on the basis of IsPETase (amino acid sequence of IsPETase set forth in SEQ ID NO: 21) by replacing the amino acid sequence set forth in SEQ ID NO: 1 with the amino acid sequence set forth in SEQ ID NO: 10, replacing the amino acid sequence set forth in SEQ ID NO: 2 with the amino acid sequence set forth in SEQ ID NO: 12, replacing the amino acid sequence set forth in SEQ ID NO: 3 with the amino acid sequence set forth in SEQ ID NO: 14, replacing the amino acid sequence set forth in SEQ ID NO: 4 with the amino acid sequence set forth in SEQ ID NO: 15, replacing the amino acid sequence set forth in SEQ ID NO: 5 with the amino acid sequence set forth in SEQ ID NO: 16, replacing the amino acid sequence set forth in SEQ ID NO: 7 with the amino acid sequence set forth in SEQ ID NO: 19, and replacing the amino acid sequence set forth in SEQ ID NO: 8 with the amino acid sequence set forth in SEQ ID NO: 20. The nucleotide sequence encoding the IsPETase-8×Chimera recombinase was set forth in SEQ ID NO.27.

### Example 7.

The example of the present application provides an IsPETase-8×Chimera recombinase with a high activity and thermal stability. The IsPETase-8×Chimera recombinase was obtained on the basis of IsPETase (amino acid sequence of IsPETase set forth in SEQ ID NO: 21) by replacing the amino acid sequence set forth in SEQ ID NO: 2 with the amino acid sequence set forth in SEQ ID NO: 11, replacing the amino acid sequence set forth in SEQ ID NO: 3 with the amino acid sequence set forth in SEQ ID NO: 14, replacing the amino acid sequence set forth in SEQ ID NO: 4 with the amino acid sequence set forth in SEQ ID NO: 15, replacing the amino acid sequence set forth in SEQ ID NO: 5 with the amino acid sequence set forth in SEQ ID NO: 16, replacing the amino acid sequence set forth in SEQ ID NO: 6 with the amino acid sequence set forth in SEQ ID NO: 18, replacing the amino acid sequence set forth in SEQ ID NO: 7 with the amino acid sequence set forth in SEQ ID NO: 19, and replacing the amino acid sequence set forth in SEQ ID NO: 8 with the amino acid sequence set forth in SEQ ID NO: 20. The nucleotide sequence encoding the IsPETase-8×Chimera recombinase was set forth in SEQ ID NO.28.

### Example 8.

The example of the present application provides an IsPETase-8×Chimera recombinase with a high activity and thermal stability. The IsPETase-8×Chimera recombinase was obtained on the basis of IsPETase (amino acid sequence of IsPETase set forth in SEQ ID NO: 21) by replacing the amino acid sequence set forth in SEQ ID NO: 2 with the amino acid sequence set forth in SEQ ID NO: 12, replacing the amino acid sequence set forth in SEQ ID NO: 3 with the amino acid sequence set forth in SEQ ID NO: 14, replacing the amino acid sequence set forth in SEQ ID NO: 4 with the amino acid sequence set forth in SEQ ID NO: 15, replacing the amino acid sequence set forth in SEQ ID NO: 5 with the amino acid sequence set forth in SEQ ID NO: 17, replacing the amino acid sequence set forth in SEQ ID NO: 6 with the amino acid sequence set forth in SEQ ID NO: 18, replacing the amino acid sequence set forth in SEQ ID NO: 7 with the amino acid sequence set forth in SEQ ID NO: 19, and replacing the amino acid sequence set forth in SEQ ID NO: 8 with the amino acid sequence set forth in SEQ ID NO: 20. The nucleotide sequence encoding the IsPETase-8×Chimera recombinase was set forth in SEQ ID NO.29.

### Example 9.

The example of the present application provides an IsPETase-8×Chimera recombinase with a high activity and thermal stability. The IsPETase-8×Chimera recombinase was obtained on the basis of IsPETase (amino acid sequence of IsPETase set forth in SEQ ID NO: 21) by replacing the amino acid sequence set forth in SEQ ID NO: 1 with the amino acid sequence set forth in SEQ ID NO: 9, replacing the amino acid sequence set forth in SEQ ID NO: 2 with the amino acid sequence set forth in SEQ ID NO: 12, replacing the amino acid sequence set forth in SEQ ID NO: 3 with the amino acid sequence set forth in SEQ ID NO: 14, replacing the amino acid sequence set forth in SEQ ID NO: 4 with the amino acid sequence set forth in SEQ ID NO: 15, replacing the amino acid sequence set forth in SEQ ID NO: 5 with the amino acid sequence set forth in SEQ ID NO: 17, replacing the amino acid sequence set forth in SEQ ID NO: 7 with the amino acid sequence set forth in SEQ ID NO: 19, and replacing the amino acid sequence set forth in SEQ ID NO: 8 with the amino acid sequence set forth in SEQ ID NO: 20. The nucleotide sequence encoding the IsPETase-8×Chimera recombinase was set forth in SEQ ID NO.30.

### Example 10.

The example of the present application provides an IsPETase-8×Chimera recombinase with a high activity and thermal stability. The IsPETase-8×Chimera recombinase was obtained on the basis of IsPETase (amino acid sequence of IsPETase set forth in SEQ ID NO: 21) by replacing the amino acid sequence set forth in SEQ ID NO: 1 with the amino acid sequence set forth in SEQ ID NO: 9, replacing the amino acid sequence set forth in SEQ ID NO: 2 with the amino acid sequence set forth in SEQ ID NO: 12, replacing the amino acid sequence set forth in SEQ ID NO: 3 with the amino acid sequence set forth in SEQ ID NO: 14, replacing the amino acid sequence set forth in SEQ ID NO: 4 with the amino acid sequence set forth in SEQ ID NO: 15, replacing the amino acid sequence set forth in SEQ ID NO: 5 with the amino acid sequence set forth in SEQ ID NO: 16, and replacing the amino acid sequence set forth in SEQ ID NO: 7 with the amino acid sequence set forth in SEQ ID NO: 19. The nucleotide sequence encoding the IsPETase-8×Chimera recombinase was set forth in SEQ ID NO.31.

### Example 11.

The example of the present application provides an IsPETase-8×Chimera recombinase with a high activity and thermal stability. The IsPETase-8×Chimera recombinase was obtained on the basis of IsPETase (amino acid sequence of IsPETase set forth in SEQ ID NO: 21) by replacing the amino acid sequence set forth in SEQ ID NO: 1 with the amino acid sequence set forth in SEQ ID NO: 10, replacing the amino acid sequence set forth in SEQ ID NO: 2 with the amino acid sequence set forth in SEQ ID NO: 11, replacing the amino acid sequence set forth in SEQ ID NO: 3 with the amino acid sequence set forth in SEQ ID NO: 14, replacing the amino acid sequence set forth in SEQ ID NO: 4 with the amino acid sequence set forth in SEQ ID NO: 15, replacing the amino acid sequence set forth in SEQ ID NO: 5 with the amino acid sequence set forth in SEQ ID NO: 16, replacing the amino acid sequence set forth in SEQ ID NO: 6 with the amino acid sequence set forth in SEQ ID NO: 18, replacing the amino acid sequence set forth in SEQ ID NO: 7 with the amino acid sequence set forth in SEQ ID NO: 19, and replacing the amino acid sequence set forth in SEQ ID NO: 8 with the amino acid sequence set forth in SEQ ID NO: 20. The nucleotide sequence encoding the IsPETase-8×Chimera recombinase was set forth in SEQ ID NO.32.

### Example 12.

The example of the present application provides an IsPETase-8×Chimera recombinase with a high activity and thermal stability. The IsPETase-8×Chimera recombinase was obtained on the basis of IsPETase (amino acid sequence of IsPETase set forth in SEQ ID NO: 21) by replacing the amino acid sequence set forth in SEQ ID NO: 1 with the amino acid sequence set forth in SEQ ID NO: 9, replacing the amino acid sequence set forth in SEQ ID NO: 2 with the amino acid sequence set forth in SEQ ID NO: 12, replacing the amino acid sequence set forth in SEQ ID NO: 3 with the amino acid sequence set forth in SEQ ID NO: 13, replacing the amino acid sequence set forth in SEQ ID NO: 4 with the amino acid sequence set forth in SEQ ID NO: 15, replacing the amino acid sequence set forth in SEQ ID NO: 5 with the amino acid sequence set forth in SEQ ID NO: 16, replacing the amino acid sequence set forth in SEQ ID NO: 6 with the amino acid sequence set forth in SEQ ID NO: 18, replacing the amino acid sequence set forth in SEQ ID NO: 7 with the amino acid sequence set forth in SEQ ID NO: 19, and replacing the amino acid sequence set forth in SEQ ID NO: 8 with the amino acid sequence set forth in SEQ ID NO: 20. The nucleotide sequence encoding the IsPETase-8×Chimera recombinase was set forth in SEQ ID NO.33.

### Example 13.

The example of the present application provides an IsPETase-8×Chimera recombinase with a high activity and thermal stability. The IsPETase-8×Chimera recombinase was obtained on the basis of IsPETase (amino acid sequence of IsPETase set forth in SEQ ID NO: 21) by replacing the amino acid sequence set forth in SEQ ID NO: 1 with the amino acid sequence set forth in SEQ ID NO: 10, replacing the amino acid sequence set forth in SEQ ID NO: 2 with the amino acid sequence set forth in SEQ ID NO: 12, replacing the amino acid sequence set forth in SEQ ID NO: 3 with the amino acid sequence set forth in SEQ ID NO: 14, replacing the amino acid sequence set forth in SEQ ID NO: 4 with the amino acid sequence set forth in SEQ ID NO: 15, replacing the amino acid sequence set forth in SEQ ID NO: 5 with the amino acid sequence set forth in SEQ ID NO: 17, replacing the amino acid sequence set forth in SEQ ID NO: 6 with the amino acid sequence set forth in SEQ ID NO: 18, replacing the amino acid sequence set forth in SEQ ID NO: 7 with the amino acid sequence set forth in SEQ ID NO: 19, and replacing the amino acid sequence set forth in SEQ ID NO: 8 with the amino acid sequence set forth in SEQ ID NO: 20. The nucleotide sequence encoding the IsPETase-8×Chimera recombinase was set forth in SEQ ID NO.34.

### Example 14.

The example of the present application provides an IsPETase-8×Chimera recombinase with a high activity and thermal stability. The IsPETase-8×Chimera recombinase was obtained on the basis of IsPETase (amino acid sequence of IsPETase set forth in SEQ ID NO: 21) by replacing the amino acid sequence set forth in SEQ ID NO: 1 with the amino acid sequence set forth in SEQ ID NO: 9, replacing the amino acid sequence set forth in SEQ ID NO: 2 with the amino acid sequence set forth in SEQ ID NO: 11, replacing the amino acid sequence set forth in SEQ ID NO: 3 with the amino acid sequence set forth in SEQ ID NO: 14, replacing the amino acid sequence set forth in SEQ ID NO: 4 with the amino acid sequence set forth in SEQ ID NO: 15, replacing the amino acid sequence set forth in SEQ ID NO: 5 with the amino acid sequence set forth in SEQ ID NO: 17, replacing the amino acid sequence set forth in SEQ ID NO: 6 with the amino acid sequence set forth in SEQ ID NO: 18, replacing the amino acid sequence set forth in SEQ ID NO: 7 with the amino acid sequence set forth in SEQ ID NO: 19, and replacing the amino acid sequence set forth in SEQ ID NO: 8 with the amino acid sequence set forth in SEQ ID NO: 20. The nucleotide sequence encoding the IsPETase-8×Chimera recombinase was set forth in SEQ ID NO.35.

### Example 15.

The example of the present application provides an IsPETase-8×Chimera recombinase with a high activity and thermal stability. The IsPETase-8×Chimera recombinase was obtained on the basis of IsPETase (amino acid sequence of IsPETase set forth in SEQ ID NO: 21) by replacing the amino acid sequence set forth in SEQ ID NO: 1 with the amino acid sequence set forth in SEQ ID NO: 9, replacing the amino acid sequence set forth in SEQ ID NO: 2 with the amino acid sequence set forth in SEQ ID NO: 12, replacing the amino acid sequence set forth in SEQ ID NO: 3 with the amino acid sequence set forth in SEQ ID NO: 14, replacing the amino acid sequence set forth in SEQ ID NO: 4 with the amino acid sequence set forth in SEQ ID NO: 15, replacing the amino acid sequence set forth in SEQ ID NO: 5 with the amino acid sequence set forth in SEQ ID NO: 16, replacing the amino acid sequence set forth in SEQ ID NO: 7 with the amino acid sequence set forth in SEQ ID NO: 19, and replacing the amino acid sequence set forth in SEQ ID NO: 8 with the amino acid sequence set forth in SEQ ID NO: 20. The nucleotide sequence encoding the IsPETase-8×Chimera recombinase was set forth in SEQ ID NO.36.

### Example 16.

The example of the present application provides an IsPETase-8×Chimera recombinase with a high activity and thermal stability. The IsPETase-8×Chimera recombinase was obtained on the basis of IsPETase (amino acid sequence of IsPETase set forth in SEQ ID NO: 21) by replacing the amino acid sequence set forth in SEQ ID NO: 2 with the amino acid sequence set forth in SEQ ID NO: 12, replacing the amino acid sequence set forth in SEQ ID NO: 3 with the amino acid sequence set forth in SEQ ID NO: 14, replacing the amino acid sequence set forth in SEQ ID NO: 4 with the amino acid sequence set forth in SEQ ID NO: 15, replacing the amino acid sequence set forth in SEQ ID NO: 5 with the amino acid sequence set forth in SEQ ID NO: 16, replacing the amino acid sequence set forth in SEQ ID NO: 6 with the amino acid sequence set forth in SEQ ID NO: 18, replacing the amino acid sequence set forth in SEQ ID NO: 7 with the amino acid sequence set forth in SEQ ID NO: 19, and replacing the amino acid sequence set forth in SEQ ID NO: 8 with the amino acid sequence set forth in SEQ ID NO: 20. The nucleotide sequence encoding the IsPETase-8×Chimera recombinase was set forth in SEQ ID NO.37.

### Example 17.

The example of the present application provides an IsPETase-8×Chimera recombinase with a high activity and thermal stability. The IsPETase-8×Chimera recombinase was obtained on the basis of IsPETase (amino acid sequence of IsPETase set forth in SEQ ID NO: 21) by replacing the amino acid sequence set forth in SEQ ID NO: 1 with the amino acid sequence set forth in SEQ ID NO: 10, replacing the amino acid sequence set forth in SEQ ID NO: 2 with the amino acid sequence set forth in SEQ ID NO: 12, replacing the amino acid sequence set forth in SEQ ID NO: 3 with the amino acid sequence set forth in SEQ ID NO: 14, replacing the amino acid sequence set forth in SEQ ID NO: 4 with the amino acid sequence set forth in SEQ ID NO: 15, replacing the amino acid sequence set forth in SEQ ID NO: 5 with the amino acid sequence set forth in SEQ ID NO: 16, replacing the amino acid sequence set forth in SEQ ID NO: 6 with the amino acid sequence set forth in SEQ ID NO: 18, replacing the amino acid sequence set forth in SEQ ID NO: 7 with the amino acid sequence set forth in SEQ ID NO: 19, and replacing the amino acid sequence set forth in SEQ ID NO: 8 with the amino acid sequence set forth in SEQ ID NO: 20. The nucleotide sequence encoding the IsPETase-8×Chimera recombinase was set forth in SEQ ID NO.38.

### Example 18.

The example of the present application provides an IsPETase-8×Chimera recombinase with a high activity and thermal stability. The IsPETase-8×Chimera recombinase was obtained on the basis of IsPETase (amino acid sequence of IsPETase set forth in SEQ ID NO: 21) by replacing the amino acid sequence set forth in SEQ ID NO: 1 with the amino acid sequence set forth in SEQ ID NO: 9, replacing the amino acid sequence set forth in SEQ ID NO: 2 with the amino acid sequence set forth in SEQ ID NO: 11, replacing the amino acid sequence set forth in SEQ ID NO: 3 with the amino acid sequence set forth in SEQ ID NO: 14, replacing the amino acid sequence set forth in SEQ ID NO: 4 with the amino acid sequence set forth in SEQ ID NO: 15, replacing the amino acid sequence set forth in SEQ ID NO: 5 with the amino acid sequence set forth in SEQ ID NO: 16, replacing the amino acid sequence set forth in SEQ ID NO: 6 with the amino acid sequence set forth in SEQ ID NO: 18, replacing the amino acid sequence set forth in SEQ ID NO: 7 with the amino acid sequence set forth in SEQ ID NO: 19, and replacing the amino acid sequence set forth in SEQ ID NO: 8 with the amino acid sequence set forth in SEQ ID NO: 20. The nucleotide sequence encoding the IsPETase-8×Chimera recombinase was set forth in SEQ ID NO.39.

### Example 19.

The example of the present application provides an IsPETase-8×Chimera recombinase with a high activity and thermal stability. The IsPETase-8×Chimera recombinase was obtained on the basis of IsPETase (amino acid sequence of IsPETase set forth in SEQ ID NO: 21) by replacing the amino acid sequence set forth in SEQ ID NO: 1 with the amino acid sequence set forth in SEQ ID NO: 9, replacing the amino acid sequence set forth in SEQ ID NO: 2 with the amino acid sequence set forth in SEQ ID NO: 12, replacing the amino acid sequence set forth in SEQ ID NO: 3 with the amino acid sequence set forth in SEQ ID NO: 14, replacing the amino acid sequence set forth in SEQ ID NO: 4 with the amino acid sequence set forth in SEQ ID NO: 15, replacing the amino acid sequence set forth in SEQ ID NO: 5 with the amino acid sequence set forth in SEQ ID NO: 17, replacing the amino acid sequence set forth in SEQ ID NO: 6 with the amino acid sequence set forth in SEQ ID NO: 18, replacing the amino acid sequence set forth in SEQ ID NO: 7 with the amino acid sequence set forth in SEQ ID NO: 19, and replacing the amino acid sequence set forth in SEQ ID NO: 8 with the amino acid sequence set forth in SEQ ID NO: 20. The nucleotide sequence encoding the IsPETase-8×Chimera recombinase was set forth in SEQ ID NO.40.

### Example 20.

The example of the present application provides an IsPETase-8×Chimera recombinase with a high activity and thermal stability. The IsPETase-8×Chimera recombinase was obtained on the basis of IsPETase (amino acid sequence of IsPETase set forth in SEQ ID NO: 21) by replacing the amino acid sequence set forth in SEQ ID NO: 1 with the amino acid sequence set forth in SEQ ID NO: 9, replacing the amino acid sequence set forth in SEQ ID NO: 2 with the amino acid sequence set forth in SEQ ID NO: 12, replacing the amino acid sequence set forth in SEQ ID NO: 3 with the amino acid sequence set forth in SEQ ID NO: 14, replacing the amino acid sequence set forth in SEQ ID NO: 4 with the amino acid sequence set forth in SEQ ID NO: 15, replacing the amino acid sequence set forth in SEQ ID NO: 5 with the amino acid sequence set forth in SEQ ID NO: 16, replacing the amino acid sequence set forth in SEQ ID NO: 6 with the amino acid sequence set forth in SEQ ID NO: 18, replacing the amino acid sequence set forth in SEQ ID NO: 7 with the amino acid sequence set forth in SEQ ID NO: 19, and replacing the amino acid sequence set forth in SEQ ID NO: 8 with the amino acid sequence set forth in SEQ ID NO: 20. The nucleotide sequence encoding the IsPETase-8×Chimera recombinase was set forth in SEQ ID NO.41.

### Example 21.

The example of the present application provides an application of the IsPETase-8×Chimera recombinases in Examples 1 to 20 in hydrolyzing PET: a PET film (diameter (φ) being 6 millimeters, mm) was added to a glycine-sodium hydroxide buffer with a pH of 8.0 to 10.0 and a concentration of 50 mM; the IsPETase-8×Chimera recombinase (in Example 1 2, 3,..., or 20) was added to the glycine-sodium hydroxide buffer to achieve a final concentration of 400 nM to 600 nM to incubate (hydrolyze) for 1 to 3 days at a temperature of 30°C to 50°C and a rotation speed of 100 rpm to 300 rpm.

### Test Example 1.

The test example investigated the Tm value of the IsPETase-8×Chimera recombinases in Examples 1 to 20 and the degradation (hydrolyzation) effect on PET films (φ=6 mm).

The Tm value was measured using a Quantitative Real-time Polymerase Chain Reaction, Q-PCR, instrument.

The degradation effect on PET film (φ=6 mm) was performed according to the following method: a PET film (φ=6 mm) was added to a glycine-sodium hydroxide buffer with a pH of 9.0 and a concentration of 50 mM; the IsPETase-8×Chimera recombinase (in Examples 1 to 20) was added to the glycine-sodium hydroxide buffer to achieve a final concentration of 500 nM to incubate (hydrolyze) for 1 day at a temperature of 40°C to 60°C and a rotation speed of 250 rpm. The TPA production in the reaction solution obtained after the degradation of PET was detected by High Performance Liquid Chromatography, HPLC. The detection conditions of HPLC were: ultraviolet detector, characteristic absorption peak at 240 nanometers, nm, ZORBAX Eclipse Plus C18 reversed-phase column (filler particle size of 5 µm, size of 250 mm×4.6 mm), mobile phase A being a 0.1% formic acid aqueous solution, mobile phase B being acetonitrile, the volume ratio of mobile phase B in the total mobile phase rising from 5% to 70% within 20 minutes, min, column temperature being 30°C, injection volume being 10 microliters, µL, and flow rate being 0.8 milliliters per minute, mL/min.

The Tm values and TPA production were shown in Table 1.

**Table 1 The Tm values and TPA production of the IsPETase-8×Chimera recombinases in Examples 1 to 20**

| Examples | SEQ ID NO. | | | | | | | | Tm | Degradation effect on PET | |
|---|---|---|---|---|---|---|---|---|---|---|---|
| | | | | | | | | | | TPA production (µM) | Degradation temperature |
| 1 | 9 | 11 | 13 | 15 | 16 | 18 | 19 | 20 | 78.17°C | 4095.82 | 50 °C |
| 2 | 9 | 11 | 14 | 15 | 16 | 6 | 19 | 20 | 78.25°C | 4609.33 | 50 °C |
| 3 | 10 | 11 | 14 | 15 | 17 | 18 | 19 | 20 | 78.29°C | 4408.21 | 40 °C |
| 4 | 9 | 12 | 14 | 15 | 17 | 18 | 19 | 8 | 78.62°C | 4105.10 | 50 °C |
| 5 | 9 | 12 | 13 | 15 | 17 | 18 | 19 | 20 | 78.64°C | 4278.37 | 60 °C |
| 6 | 10 | 12 | 14 | 15 | 16 | 6 | 19 | 20 | 78.76°C | 4643.21 | 60 °C |
| 7 | 1 | 11 | 14 | 15 | 16 | 18 | 19 | 20 | 78.93°C | 4216.35 | 40 °C |
| 8 | 1 | 12 | 14 | 15 | 17 | 18 | 19 | 20 | 79.04°C | 3854.70 | 50 °C |
| 9 | 9 | 12 | 14 | 15 | 17 | 6 | 19 | 20 | 79.19°C | 3907.70 | 40 °C |
| 10 | 9 | 12 | 14 | 15 | 16 | 18 | 19 | 8 | 79.30°C | 4453.71 | 60 °C |
| 11 | 10 | 11 | 14 | 15 | 16 | 18 | 19 | 20 | 79.50°C | 4780.30 | 50 °C |
| 12 | 9 | 12 | 13 | 15 | 16 | 18 | 19 | 20 | 79.66°C | 4856.49 | 60 °C |
| 13 | 10 | 12 | 14 | 15 | 17 | 18 | 19 | 20 | 79.70°C | 4679.64 | 60 °C |
| 14 | 9 | 11 | 14 | 15 | 17 | 18 | 19 | 20 | 79.78°C | 4308.71 | 40 °C |
| 15 | 9 | 12 | 14 | 15 | 16 | 6 | 19 | 20 | 80.12°C | 4633.74 | 50 °C |
| 16 | 1 | 12 | 14 | 15 | 16 | 18 | 19 | 20 | 80.39°C | 4570.76 | 50 °C |
| 17 | 10 | 12 | 14 | 15 | 16 | 18 | 19 | 20 | 80.89°C | 4032.69 | 40 °C |
| 18 | 9 | 11 | 14 | 15 | 16 | 18 | 19 | 20 | 81.23°C | 4823.61 | 60 °C |
| 19 | 9 | 12 | 14 | 15 | 17 | 18 | 19 | 20 | 81.28°C | 3747.90 | 40 °C |
| 20 | 9 | 12 | 14 | 15 | 16 | 18 | 19 | 20 | 82.71°C | 4111.56 | 50 °C |

The present test example also investigated the Tm and TPA production of wild-type PET hydrolases IsPETase, TfCut2 and LCC in the same manner, and the results were as shown in Table 2.

**Table 2 The Tm and TPA production of wild-type PET hydrolases IsPETase, TfCut2 and LCC**

| | | TPA production (µM) | | | | |
|---|---|---|---|---|---|---|
| | Tm | 40°C | 50°C | 60°C | 70°C | 80°C |
| IsPETase | 46.7°C | 216.78 | 32.33 | / | / | / |
| TfCut2 | 72.7°C | 207.81 | 1266.49 | 3435.03 | 166.94 | 24.03 |
| LCC | 86.6°C | 1085.35 | 2299.36 | 3701.64 | 2405.92 | 250.05 |

### Example 22.

The example of the present application provides a recombinant plasmid containing a gene encoding the PET hydrolase IsPETase-8×Chimera recombinase in Examples 1 to 20 and a construction method therefor, and the construction method includes:
Step 1, the construction of recombinant plasmid pET-22b-IsPETase-cSP, recombinant plasmid pET-22b-TfCut2-cSP and recombinant plasmid pET-22b-LCC-cSP.

The construction of recombinant plasmid pET-22b-IsPETase: the gene of wild-type PET hydrolase IsPETase (SEQ ID NO: 42) was connected to a pET-22b plasmid to obtain the recombinant plasmid pET-22b-IsPETase.

The construction of recombinant plasmid pET-22b-TfCut2: the gene of wild-type PET hydrolase TfCut2 (SEQ ID NO: 43) was connected to a pET-22b plasmid to obtain the recombinant plasmid pET-22b-TfCut2.

The construction of recombinant plasmid pET-22b-LCC: the gene of wild-type PET hydrolase LCC (SEQ ID NO: 44) was connected to a pET-22b plasmid to obtain the recombinant plasmid pET-22b-LCC.

The gene of wild-type PET hydrolase IsPETase was sourced from Ideonella sakaiensis (GI:1028065175), the gene of wild-type PET hydrolase TfCut2 was sourced from Thermobifida fusca (GenBank: JN129500.1), and the gene of wild-type PET hydrolase LCC was sourced from uncultured bacterium (GenBank: HQ704839.1).

The pET-22b plasmid was commercially available.

A method for preparing the recombinant plasmid (recombinant plasmids pET-22b-IsPETase-cSP, pET-22b-TfCu2-cSP and pET-22b-LCC-cSP) includes the following specific steps:
(1), some primers were designed using the recombinant plasmids PET-22b-IsPETase, pET-22b-TfCut2 and pET-22b-LCC as templates, respectively, according to the 2×*TransStart*^{®} FastPfu PCR SuperMix kit of TransGen Biotech. The primers were as following:
   IsPETase-cSP-F: 5'-GAGATATACATATGCAGACCAACCCCTACGCCCGCGGCCCGAACC (SEQ ID No.45).
   IsPETase-cSP-R: 5'-GTAGGGGTTGGTCTGCATATGTATATCTCCTTCTTAAAGTTAAAC (SEQ ID No.46).
   TfCut2-cSP-F: 5'-GGAGATATAcatatgGCCAACCCCTACGAGCGCGGCCCCAACCCG (SEQ ID No.47).
   TfCut2-cSP-R: 5'-CTCGTAGGGGTTGGCcatatgTATATCTCCTTCTTAAAGTTAAAC (SEQ ID No.48).
   LCC-cSP-F: 5'-GGAGATATAcatatgGACGGAGTTCTCTGGCGAGTGCGAACCGCG (SEQ ID No.49).
   LCC-cSP-R: 5'-CCAGAGAACTCCGTCcatatgTATATCTCCTTCTTAAAGTTAAAC (SEQ ID No.50).
(2), PCR was performed to obtain linear vector fragments of 6.2 kilobases, kb, 6.2 kb and 6.2 kb corresponding to each template. Each of the obtained linear vector fragments corresponding to each template was mixed with a ligase to form circular recombinant plasmids (three circular recombinant plasmids were obtained). Each of the circular recombinant plasmids was transformed into a T1 competent cell respectively to obtain a recombinant plasmid pET-22b-IsPETase-cSP, a recombinant plasmid pET-22b-TfCut2-cSP and a recombinant plasmid pET-22b-LCC-cSP by ampicillin-containing medium screening, plasmid extraction and sequencing verification. A nucleotide sequence of an IsPETase-cSP (cut off signal peptide, cSP) gene in the recombinant plasmid pET-22b-IsPETase-cSP was set forth in SEQ ID NO: 51, and an amino acid sequence of an IsPETase-cSP enzyme was set forth in SEQ ID NO: 52; a nucleotide sequence of a TfCut2-cSP gene in the recombinant plasmid pET-22b-TfCut2-cSP was set forth in SEQ ID NO: 53, and an amino acid sequence of a TfCut2-cSP enzyme was set forth in SEQ ID NO: 54; a nucleotide sequence of a LCC-cSP gene in the recombinant plasmid pET-22b-LCC-cSP was set forth in SEQ ID NO: 55, and an amino acid sequence of a LCC-cSP enzyme was set forth in SEQ ID NO: 56.

The formulations of ampicillin-containing medium were: 5 grams per liter, g/L, of yeast extract, 10 g/L of tryptone, 10 g/L of sodium chloride, 15 g/L of agar powder, and 50 milligrams per liter, mg/L, of ampicillin.

The PCR reaction system and PCR reaction program were performed with reference to the requirements of the 2×*TransStart*^{®} FastPfu PCR SuperMix kit of TransGen Biotech, as shown in Table 3:

**Table 3 PCR reaction system**

| PCR reaction system (50µL) | | PCR reaction program | |
|---|---|---|---|
| Template | Variable | | 95°C, 2min |
| Forward primer (10µM) | 1µL | Cycle number being 30 to 35 | 95°C, 20 seconds, s |
| Reverse primer (10µM) | 1µL | | Tm-5°C, 20s |
| 2×*TransStart*^{®} FastPfu PCR SuperMix | 25µL | | 72°C, 4kb/min |
| Nuclease-free Water | Variable | | 72°C, 5min |

Step 2, the construction of a recombinant plasmid containing a gene encoding the IsPETase-8×Chimera recombinase (as exemplified in Example 18).

The primers required for recombination were designed and synthesized.
8×chimera 18-1-F: 5'-GAAGCTGTGGGTGGATAACGACACCCGCTACACCCAGTTCCTCTG (SEQ ID No.57).
8×chimera 18-1-R: 5'-GCTCCACGTACACCTTGCTGATGGAGCTCGGCAGG (SEQ ID No.58).
8×chimera 18-2-F: 5'-CCGAGCTCCATCAGCAAGGTGTACGTGGAGCTCGAC (SEQ ID No.59).
8×chimera 18-2-R: 5'-CAGAGGAACTGGGTGTAGCGGGTGTCGTTATCCACCCACAGCTTC (SEQ ID No.60).

A recombinant plasmid containing a gene encoding the IsPETase-8×Chimera recombinase in Example 18 was constructed by PCR (the amplification system was the same as that in Table 3) and by seamless ligation as required for a seamless recombination kit. The process mainly included three steps: (1) PCR was performed (PCR reaction program: 95°C for 2 min; 95°C for 20 s, 76°C for 20 s, 72°C for 5 min, 30 cycles; 72°C for 5 min) using the recombinant plasmid pET-22b-TfCut2-cSP as a template, and using 8×chimera 18-1-F and 8×chimera 18-1-R as a forward (upstream) primer and a reverse (downstream) primer, respectively; DMT enzyme digestion, nucleic acid electrophoresis and degumming recovery were performed on the PCR product to obtain a first group of purified gene fragments; (2) PCR was performed (PCR reaction program: 95°C for 2 min; 95°C for 20 s, 76°C for 20 s, 72°C for 5 min, 30 cycles; 72°C for 5 min) using the recombinant plasmid pET-22b-LCC-cSP as a template, 8×chimera 18-2-F and 8×chimera 18-2-R as a forward primer and a reverse primer, respectively; DMT enzyme digestion, nucleic acid electrophoresis and degumming recovery were performed on the PCR product to obtain a second group of purified gene fragments; and (3) the first group of purified gene fragments and the second group of purified gene fragments were ligated with a seamless recombination ligase to transform into a T1 competent cell to obtain the recombinant plasmid containing the gene encoding the IsPETase-8×Chimera recombinase in Example 18 by means of ampicillin-containing medium screening, plasmid extraction and sequencing verification. The nucleotide sequence of a gene encoding the IsPETase-8×Chimera 18 recombinase was set forth in SEQ ID No. 39.

The formulations of ampicillin-containing medium were: 5 g/L of yeast extract, 10 g/L of tryptone, 10 g/L of sodium chloride, 15 g/L of agar powder, and 50 mg/L of ampicillin. The seamless recombination ligase was a seamless cloning enzyme, and 2X MultiF Seamless Assembly Mix produced by ABclonal was used in the present application.

When constructing the IsPETase-8×Chimera recombinases in other examples, the primers required for recombination corresponding to each recombinase (see Table 4) were designed and synthesized, and the recombinant plasmids containing genes encoding the respective recombinases were constructed by seamless ligation and PCR using the recombinant plasmid pET-22b-IsPETase-cSP, the recombinant plasmid pET-22b-TfCut2-cSP and the recombinant plasmid pET-22b-LCC-cSP in step 1 according to the requirements of the seamless recombination kit.

**Table 4 Primers and templates corresponding to IsPETase-8×Chimera recombinases in Examples 1 to 20**

| Exampl es | Template 1 | Forward primer 1 | Reverse primer 1 |
|---|---|---|---|
| 1 | pET-22b-TfCut2-cSP | GAAGCTGTGGGTGGATAACGA CACCCGCTACACCCAGTTCCTC TG | CCGGCGACATGGCGATCCCGCC GTAGGTGTTGTTCTCCCGCGGG |
| | Template 2 | Forward primer 2 | Reverse primer 2 |
| | pET-22b-LCC-cSP | CCCGCGGAGAACAACACCTA CGGCGGGATCGCCATGTCGCCG G | GATGGTGTCGATGGTGATGACG ACGAAGCCGTGCGATGCCAAC CGC |
| | Template 3 | Forward primer 3 | Reverse primer 3 |
| | pET-22b-TfCut2-cSP | CGGTTGGCATCGCACGGCTTCG TCGTCATCACCATCGACACCAT C | GCTCCACGTACACCTTGCTGAT GGAGCTCGGCAGG |
| | Template 4 | Forward primer 4 | Reverse primer 4 |
| | pET-22b-LCC-cSP | CCGAGCTCCATCAGCAAGGTGT ACGTGGAGCTCGAC | CAGAGGAACTGGGTGTAGCGG GTGTCGTTATCCACCCACAGCT TC |
| 2 | Template 1 | Forward primer 1 | Reverse primer 1 |
| | pET-22b-TfCut2-cSP | GAAGCTGTGGGTGGATAACGA CACCCGCTACACCCAGTTCCTC TG | cgcaggcgaagatcagCGTCGGCACGG TGACGCTGC |
| | Template 2 | Forward primer 2 | Reverse primer 2 |
| | pET-22b-IsPETase-cSP | CGTCACCGTGCCGACGctgatcttcg cctgcgagaacg | CTCCACGTACACCTTcgcattgcgcg acatgctgtc |
| | Template 3 | Forward primer 3 | Reverse primer 3 |
| | pET-22b-LCC-cSP | catgtcgcgcaatgcgAAGGTGTACGT GGAGCTCGACAATG | CAGAGGAACTGGGTGTAGCGG GTGTCGTTATCCACCCACAGCT TC |
| 3 | Template 1 | Forward primer 1 | Reverse primer 1 |
| | pET-22b-LCC-cSP | CCACCTGCCCGTTCctcgagCACC ACCACCACCACCACTG | GGGAGACGTTCTCCTCGCTGAC CGAGAACGGCCCGTCGGCCGT G |
| | Template 2 | Forward primer 2 | Reverse primer 2 |
| | pET-22b-TfCut2-cSP | CACGGCCGACGGGCCGTTCTCG GTCAGCGAGGAGAACGTCTCC C | GCCGCCGCCCATCGAGTGGCCC ATGACCGCCAGTCGG |
| | Template 3 | Forward primer 3 | Reverse primer 3 |
| | pET-22b-LCC-cSP | GCGGTCATGGGCCACTCGATGG GCGGCGGCGGGAC | CGAGGTCGGCCCCGATGATCA GCACCGGTACCGACGTGTTGAA CG |
| | Template 4 | Forward primer 4 | Reverse primer 4 |
| | pET-22b-TfCut2-cSP | GTTCAACACGTCGGTACCGGTG CTGATCATCGGGGCCGACCTCG | GCTCCACGTACACCTTGCTGAT GGAGCTCGGCAGG |
| | Template 5 | Forward primer 5 | Reverse primer 5 |
| | pET-22b-LCC-cSP | CCGAGCTCCATCAGCAAGGTGT ACGTGGAGCTCGAC | CAGAGGAACTGGGTGTAGCGG GTGTCGTTATCCACCCACAGCT TC |
| | Template 6 | Forward primer 6 | Reverse primer 6 |
| | pET-22b-TfCut2-cSP | GAAGCTGTGGGTGGATAACGA CACCCGCTACACCCAGTTCCTC TG | GTGGTGGTGctcgagGAACGGGC AGGTGGAGCGG |
| 4 | Template 1 | Forward primer 1 | Reverse primer 1 |
| | pET-22b-TfCut2-cSP | cgcgaactgcagcctcgagCACCACCAC CACCACCACTG | CCGCGAAACGGTGTAGGTTGCC ACGGAGAAGGGGCCGCTG |
| | Template 2 | Forward primer 2 | Reverse primer 2 |
| | pET-22b-LCC-cSP | CAGCGGCCCCTTCTCCGTGGCA ACCTACACCGTTTCGC | AGATCGCCACCGCACCGAAGG TCAGCGAGGTGCCTGTGGG |
| | Template 3 | Forward primer 3 | Reverse primer 3 |
| | pET-22b-TfCut2-cSP | CACCTCGCTGACCTTCGGTGCG GTGGCGATCTCCCCCGGC | GCCGCCGCCCATCGAGTGGCCC ATGACCGCCAGTCGG |
| | Template 4 | Forward primer 4 | Reverse primer 4 |
| | pET-22b-LCC-cSP | GCGGTCATGGGCCACTCGATGG GCGGCGGCGGGAC | CGAGGTCGGCCCCGATGATCA GCACCGGTACCGACGTGTTGAA CG |
| | Template 5 | Forward primer 5 | Reverse primer 5 |
| | pET-22b-TfCut2-cSP | GTTCAACACGTCGGTACCGGTG CTGATCATCGGGGCCGACCTCG | GCTCCACGTACACCTTGCTGAT GGAGCTCGGCAGG |
| | Template 6 | Forward primer 6 | Reverse primer 6 |
| | pET-22b-LCC-cSP | CCGAGCTCCATCAGCAAGGTGT ACGTGGAGCTCGAC | ggtggagtagcgcgtGTCGTTATCCAC CCACAGCTTC |
| | Template 7 | Forward primer 7 | Reverse primer 7 |
| | pET-22b-IsPETase-cSP | GTGGGTGGATAACGACacgcgctac tccaccttcg | CAGTGGTGGTGGTGGTGGTGctc gaggctgcagttcgcggtgcgg |
| 5 | Template 1 | Forward primer 1 | Reverse primer 1 |
| | pET-22b-TfCut2-cSP | GAAGCTGTGGGTGGATAACGA CACCCGCTACACCCAGTTCCTC TG | CCGCGAAACGGTGTAGGTTGCC ACGGAGAAGGGGCCGCTG |
| | Template 2 | Forward primer 2 | Reverse primer 2 |
| | pET-22b-LCC-cSP | CAGCGGCCCCTTCTCCGTGGCA ACCTACACCGTTTCGC | GATGGTGTCGATGGTGATGACG ACGAAGCCGTGCGATGCCAAC CGC |
| | Template 3 | Forward primer 3 | Reverse primer 3 |
| | pET-22b-TfCut2-cSP | CGGTTGGCATCGCACGGCTTCG TCGTCATCACCATCGACACCAT C | GCCGCCGCCCATCGAGTGGCCC ATGACCGCCAGTCGG |
| | Template 4 | Forward primer 4 | Reverse primer 4 |
| | pET-22b-LCC-cSP | GCGGTCATGGGCCACTCGATGG GCGGCGGCGGGAC | CGAGGTCGGCCCCGATGATCA GCACCGGTACCGACGTGTTGAA CG |
| | Template 5 | Forward primer 5 | Reverse primer 5 |
| | pET-22b-TfCut2-cSP | GTTCAACACGTCGGTACCGGTG CTGATCATCGGGGCCGACCTCG | GCTCCACGTACACCTTGCTGAT GGAGCTCGGCAGG |
| | Template 6 | Forward primer 6 | Reverse primer 6 |
| | pET-22b-LCC-cSP | CCGAGCTCCATCAGCAAGGTGT ACGTGGAGCTCGAC | CAGAGGAACTGGGTGTAGCGG GTGTCGTTATCCACCCACAGCT TC |
| 6 | Template 1 | Forward primer 1 | Reverse primer 1 |
| | pET-22b-LCC-cSP | CCACCTGCCCGTTCctcgagCACC ACCACCACCACCACTG | AGATCGCCACCGCACCGAAGG TCAGCGAGGTGCCTGTGGG |
| | Template 2 | Forward primer 2 | Reverse primer 2 |
| | pET-22b-TfCut2-cSP | CACCTCGCTGACCTTCGGTGCG GTGGCGATCTCCCCCGGC | cgcaggcgaagatcagCGTCGGCACGG TGACGCTGC |
| | Template 3 | Forward primer 3 | Reverse primer 3 |
| | pET-22b-IsPETase-cSP | CGTCACCGTGCCGACGctgatcttcg cctgcgagaacg | CTCCACGTACACCTTcgcattgcgcg acatgctgtc |
| | Template 4 | Forward primer 4 | Reverse primer 4 |
| | pET-22b-LCC-cSP | catgtcgcgcaatgcgAAGGTGTACGT GGAGCTCGACAATG | CAGAGGAACTGGGTGTAGCGG GTGTCGTTATCCACCCACAGCT TC |
| | Template 5 | Forward primer 5 | Reverse primer 5 |
| | pET-22b-TfCut2-cSP | GAAGCTGTGGGTGGATAACGA CACCCGCTACACCCAGTTCCTC TG | GTGGTGGTGctcgagGAACGGGC AGGTGGAGCGG |
| 7 | Template 1 | Forward primer 1 | Reverse primer 1 |
| | pET-22b-IsPETase-cSP | CCACCTGCCCGTTCctcgagCACC ACCACCACCACCACTG | CGTTCTCCTCGCTGACggtgaacgg gccggcgctgg |
| | Template 2 | Forward primer 2 | Reverse primer 2 |
| | pET-22b-TfCut2-cSP | cgccggcccgttcaccGTCAGCGAGGA GAACGTCTCC | GCTCCACGTACACCTTGCTGAT GGAGCTCGGCAGG |
| | Template 3 | Forward primer 3 | Reverse primer 3 |
| | pET-22b-LCC-cSP | CCGAGCTCCATCAGCAAGGTGT ACGTGGAGCTCGAC | CAGAGGAACTGGGTGTAGCGG GTGTCGTTATCCACCCACAGCT TC |
| | Template 4 | Forward primer 4 | Reverse primer 4 |
| | pET-22b-TfCut2-cSP | GAAGCTGTGGGTGGATAACGA CACCCGCTACACCCAGTTCCTC TG | GTGGTGGTGctcgagGAACGGGC AGGTGGAGCGG |
| 8 | Template 1 | Forward primer 1 | Reverse primer 1 |
| | pET-22b-IsPETase-cSP | CCACCTGCCCGTTCctcgagCACC ACCACCACCACCACTG | GGTGTAGGTTGCCACggtgaacggg ccggcgctg |
| | Template 2 | Forward primer 2 | Reverse primer 2 |
| | pET-22b-LCC-cSP | ccggcccgttcaccGTGGCAACCTACA CCGTTTCGC | AGATCGCCACCGCACCGAAGG TCAGCGAGGTGCCTGTGGG |
| | Template 3 | Forward primer 3 | Reverse primer 3 |
| | pET-22b-TfCut2-cSP | CACCTCGCTGACCTTCGGTGCG GTGGCGATCTCCCCCGGC | GCCGCCGCCCATCGAGTGGCCC ATGACCGCCAGTCGG |
| | Template 4 | Forward primer 4 | Reverse primer 4 |
| | pET-22b-LCC-cSP | GCGGTCATGGGCCACTCGATGG GCGGCGGCGGGAC | CGAGGTCGGCCCCGATGATCA GCACCGGTACCGACGTGTTGAA CG |
| | Template 5 | Forward primer 5 | Reverse primer 5 |
| | pET-22b-TfCut2-cSP | GTTCAACACGTCGGTACCGGTG CTGATCATCGGGGCCGACCTCG | GCTCCACGTACACCTTGCTGAT GGAGCTCGGCAGG |
| | Template 6 | Forward primer 6 | Reverse primer 6 |
| | pET-22b-LCC-cSP | CCGAGCTCCATCAGCAAGGTGT ACGTGGAGCTCGAC | CAGAGGAACTGGGTGTAGCGG GTGTCGTTATCCACCCACAGCT TC |
| | Template 7 | Forward primer 7 | Reverse primer 7 |
| | pET-22b-TfCut2-cSP | GAAGCTGTGGGTGGATAACGA CACCCGCTACACCCAGTTCCTC TG | GTGGTGGTGctcgagGAACGGGC AGGTGGAGCGG |
| 9 | Template 1 | Forward primer 1 | Reverse primer 1 |
| | pET-22b-TfCut2-cSP | GAAGCTGTGGGTGGATAACGA CACCCGCTACACCCAGTTCCTC TG | CCGCGAAACGGTGTAGGTTGCC ACGGAGAAGGGGCCGCTG |
| | Template 2 | Forward primer 2 | Reverse primer 2 |
| | pET-22b-LCC-cSP | CAGCGGCCCCTTCTCCGTGGCA ACCTACACCGTTTCGC | AGATCGCCACCGCACCGAAGG TCAGCGAGGTGCCTGTGGG |
| | Template 3 | Forward primer 3 | Reverse primer 3 |
| | pET-22b-TfCut2-cSP | CACCTCGCTGACCTTCGGTGCG GTGGCGATCTCCCCCGGC | GCCGCCGCCCATCGAGTGGCCC ATGACCGCCAGTCGG |
| | Template 4 | Forward primer 4 | Reverse primer 4 |
| | pET-22b-LCC-cSP | GCGGTCATGGGCCACTCGATGG GCGGCGGCGGGAC | cgttctcgcaggcgaagatcagCACCGGTA CCGACGTGTTGAACG |
| | Template 5 | Forward primer 5 | Reverse primer 5 |
| | pET-22b-IsPETase-cSP | CGTTCAACACGTCGGTACCGGT Gctgatcttcgcctgcgagaacg | CTCCACGTACACCTTcgcattgcgcg acatgctgtc |
| | Template 6 | Forward primer 6 | Reverse primer 6 |
| | pET-22b-LCC-cSP | catgtcgcgcaatgcgAAGGTGTACGT GGAGCTCGACAATG | CAGAGGAACTGGGTGTAGCGG GTGTCGTTATCCACCCACAGCT TC |
| 10 | Template 1 | Forward primer 1 | Reverse primer 1 |
| | pET-22b-TfCut2-cSP | cgcgaactgcagcctcgagCACCACCAC CACCACCACTG | CCGCGAAACGGTGTAGGTTGCC ACGGAGAAGGGGCCGCTG |
| | Template 2 | Forward primer 2 | Reverse primer 2 |
| | pET-22b-LCC-cSP | CAGCGGCCCCTTCTCCGTGGCA ACCTACACCGTTTCGC | AGATCGCCACCGCACCGAAGG TCAGCGAGGTGCCTGTGGG |
| | Template 3 | Forward primer 3 | Reverse primer 3 |
| | pET-22b-TfCut2-cSP | CACCTCGCTGACCTTCGGTGCG GTGGCGATCTCCCCCGGC | GCTCCACGTACACCTTGCTGAT GGAGCTCGGCAGG |
| | Template 4 | Forward primer 4 | Reverse primer 4 |
| | pET-22b-LCC-cSP | CCGAGCTCCATCAGCAAGGTGT ACGTGGAGCTCGAC | ggtggagtagcgcgtGTCGTTATCCAC CCACAGCTTC |
| | Template 5 | Forward primer 5 | Reverse primer 5 |
| | pET-22b-IsPETase-cSP | GTGGGTGGATAACGACacgcgctac tccaccttcg | CAGTGGTGGTGGTGGTGctc gaggctgcagttcgcggtgcgg |
| 11 | Template 1 | Forward primer 1 | Reverse primer 1 |
| | pET-22b-LCC-cSP | CCACCTGCCCGTTCctcgagCACC ACCACCACCACCACTG | GGGAGACGTTCTCCTCGCTGAC CGAGAACGGCCCGTCGGCCGT G |
| | Template 2 | Forward primer 2 | Reverse primer 2 |
| | pET-22b-TfCut2-cSP | CACGGCCGACGGGCCGTTCTCG GTCAGCGAGGAGAACGTCTCC C | GCTCCACGTACACCTTGCTGAT GGAGCTCGGCAGG |
| | Template 3 | Forward primer 3 | Reverse primer 3 |
| | pET-22b-LCC-cSP | CCGAGCTCCATCAGCAAGGTGT ACGTGGAGCTCGAC | CAGAGGAACTGGGTGTAGCGG GTGTCGTTATCCACCCACAGCT TC |
| | Template 4 | Forward primer 4 | Reverse primer 4 |
| | pET-22b-TfCut2-cSP | GAAGCTGTGGGTGGATAACGA CACCCGCTACACCCAGTTCCTC TG | GTGGTGGTGctcgagGAACGGGC AGGTGGAGCGG |
| 12 | Template 1 | Forward primer 1 | Reverse primer 1 |
| | pET-22b-TfCut2-cSP | GAAGCTGTGGGTGGATAACGA CACCCGCTACACCCAGTTCCTC TG | CCGCGAAACGGTGTAGGTTGCC ACGGAGAAGGGGCCGCTG |
| | Template 2 | Forward primer 2 | Reverse primer 2 |
| | pET-22b-LCC-cSP | CAGCGGCCCCTTCTCCGTGGCA ACCTACACCGTTTCGC | GATGGTGTCGATGGTGATGACG ACGAAGCCGTGCGATGCCAAC CGC |
| | Template 3 | Forward primer 3 | Reverse primer 3 |
| | pET-22b-TfCut2-cSP | CGGTTGGCATCGCACGGCTTCG TCGTCATCACCATCGACACCAT C | GCTCCACGTACACCTTGCTGAT GGAGCTCGGCAGG |
| | Template 4 | Forward primer 4 | Reverse primer 4 |
| | pET-22b-LCC-cSP | CCGAGCTCCATCAGCAAGGTGT ACGTGGAGCTCGAC | CAGAGGAACTGGGTGTAGCGG GTGTCGTTATCCACCCACAGCT TC |
| 13 | Template 1 | Forward primer 1 | Reverse primer 1 |
| | pET-22b-LCC-cSP | CCACCTGCCCGTTCctcgagCACC ACCACCACCACCACTG | AGATCGCCACCGCACCGAAGG TCAGCGAGGTGCCTGTGGG |
| | Template 2 | Forward primer 2 | Reverse primer 2 |
| | pET-22b-TfCut2-cSP | CACCTCGCTGACCTTCGGTGCG GTGGCGATCTCCCCCGGC | GCCGCCGCCCATCGAGTGGCCC ATGACCGCCAGTCGG |
| | Template 3 | Forward primer 3 | Reverse primer 3 |
| | pET-22b-LCC-cSP | GCGGTCATGGGCCACTCGATGG GCGGCGGCGGGAC | CGAGGTCGGCCCCGATGATCA GCACCGGTACCGACGTGTTGAA CG |
| | Template 4 | Forward primer 4 | Reverse primer 4 |
| | pET-22b-TfCut2-cSP | GTTCAACACGTCGGTACCGGTG CTGATCATCGGGGCCGACCTCG | GCTCCACGTACACCTTGCTGAT GGAGCTCGGCAGG |
| | Template 5 | Forward primer 5 | Reverse primer 5 |
| | pET-22b-LCC-cSP | CCGAGCTCCATCAGCAAGGTGT ACGTGGAGCTCGAC | CAGAGGAACTGGGTGTAGCGG GTGTCGTTATCCACCCACAGCT TC |
| | Template 6 | Forward primer 6 | Reverse primer 6 |
| | pET-22b-TfCut2-cSP | GAAGCTGTGGGTGGATAACGA CACCCGCTACACCCAGTTCCTC TG | GTGGTGGTGctcgagGAACGGGC AGGTGGAGCGG |
| 14 | Template 1 | Forward primer 1 | Reverse primer 1 |
| | pET-22b-TfCut2-cSP | GAAGCTGTGGGTGGATAACGA CACCCGCTACACCCAGTTCCTC TG | GCCGCCGCCCATCGAGTGGCCC ATGACCGCCAGTCGG |
| | Template 2 | Forward primer 2 | Reverse primer 2 |
| | pET-22b-LCC-cSP | GCGGTCATGGGCCACTCGATGG GCGGCGGCGGGAC | CGAGGTCGGCCCCGATGATCA GCACCGGTACCGACGTGTTGAA CG |
| | Template 3 | Forward primer 3 | Reverse primer 3 |
| | pET-22b-TfCut2-cSP | GTTCAACACGTCGGTACCGGTG CTGATCATCGGGGCCGACCTCG | GCTCCACGTACACCTTGCTGAT GGAGCTCGGCAGG |
| | Template 4 | Forward primer 4 | Reverse primer 4 |
| | pET-22b-LCC-cSP | CCGAGCTCCATCAGCAAGGTGT ACGTGGAGCTCGAC | CAGAGGAACTGGGTGTAGCGG GTGTCGTTATCCACCCACAGCT TC |
| 15 | Template 1 | Forward primer 1 | Reverse primer 1 |
| | pET-22b-TfCut2-cSP | GAAGCTGTGGGTGGATAACGA CACCCGCTACACCCAGTTCCTC TG | CCGCGAAACGGTGTAGGTTGCC ACGGAGAAGGGGCCGCTG |
| | Template 2 | Forward primer 2 | Reverse primer 2 |
| | pET-22b-LCC-cSP | CAGCGGCCCCTTCTCCGTGGCA ACCTACACCGTTTCGC | AGATCGCCACCGCACCGAAGG TCAGCGAGGTGCCTGTGGG |
| | Template 3 | Forward primer 3 | Reverse primer 3 |
| | pET-22b-TfCut2-cSP | CACCTCGCTGACCTTCGGTGCG GTGGCGATCTCCCCCGGC | cgcaggcgaagatcagCGTCGGCACGG TGACGCTGC |
| | Template 4 | Forward primer 4 | Reverse primer 4 |
| | pET-22b-IsPETase-cSP | CGTCACCGTGCCGACGctgatcttcg cctgcgagaacg | CTCCACGTACACCTTcgcattgcgcg acatgctgtc |
| | Template 5 | Forward primer 5 | Reverse primer 5 |
| | pET-22b-LCC-cSP | catgtcgcgcaatgcgAAGGTGTACGT GGAGCTCGACAATG | CAGAGGAACTGGGTGTAGCGG GTGTCGTTATCCACCCACAGCT TC |
| 16 | Template 1 | Forward primer 1 | Reverse primer 1 |
| | pET-22b-IsPETase-cSP | CCACCTGCCCGTTCctcgagCACC ACCACCACCACCACTG | GGTGTAGGTTGCCACggtgaacggg ccggcgctg |
| | Template 2 | Forward primer 2 | Reverse primer 2 |
| | pET-22b-LCC-cSP | ccggcccgttcaccGTGGCAACCTACA CCGTTTCGC | AGATCGCCACCGCACCGAAGG TCAGCGAGGTGCCTGTGGG |
| | Template 3 | Forward primer 3 | Reverse primer 3 |
| | pET-22b-TfCut2-cSP | CACCTCGCTGACCTTCGGTGCG GTGGCGATCTCCCCCGGC | GCTCCACGTACACCTTGCTGAT GGAGCTCGGCAGG |
| | Template 4 | Forward primer 4 | Reverse primer 4 |
| | pET-22b-LCC-cSP | CCGAGCTCCATCAGCAAGGTGT ACGTGGAGCTCGAC | CAGAGGAACTGGGTGTAGCGG GTGTCGTTATCCACCCACAGCT TC |
| | Template 5 | Forward primer 5 | Reverse primer 5 |
| | pET-22b-TfCut2-cSP | GAAGCTGTGGGTGGATAACGA CACCCGCTACACCCAGTTCCTC TG | GTGGTGGTGctcgagGAACGGGC AGGTGGAGCGG |
| 17 | Template 1 | Forward primer 1 | Reverse primer 1 |
| | pET-22b-LCC-cSP | CCACCTGCCCGTTCctcgagCACC ACCACCACCACCACTG | AGATCGCCACCGCACCGAAGG TCAGCGAGGTGCCTGTGGG |
| | Template 2 | Forward primer 2 | Reverse primer 2 |
| | pET-22b-TfCut2-cSP | CACCTCGCTGACCTTCGGTGCG GTGGCGATCTCCCCCGGC | GCTCCACGTACACCTTGCTGAT GGAGCTCGGCAGG |
| | Template 3 | Forward primer 3 | Reverse primer 3 |
| | pET-22b-LCC-cSP | CCGAGCTCCATCAGCAAGGTGT ACGTGGAGCTCGAC | CAGAGGAACTGGGTGTAGCGG GTGTCGTTATCCACCCACAGCT TC |
| | Template 4 | Forward primer 4 | Reverse primer 4 |
| | pET-22b-TfCut2-cSP | GAAGCTGTGGGTGGATAACGA CACCCGCTACACCCAGTTCCTC TG | GTGGTGGTGctcgagGAACGGGC AGGTGGAGCGG |
| 18 | Template 1 | Forward primer 1 | Reverse primer 1 |
| | pET-22b-TfCut2-cSP | GAAGCTGTGGGTGGATAACGA CACCCGCTACACCCAGTTCCTC TG | GCTCCACGTACACCTTGCTGAT GGAGCTCGGCAGG |
| | Template 2 | Forward primer 2 | Reverse primer 2 |
| | pET-22b-LCC-cSP | CCGAGCTCCATCAGCAAGGTGT ACGTGGAGCTCGAC | CAGAGGAACTGGGTGTAGCGG GTGTCGTTATCCACCCACAGCT TC |
| 19 | Template 1 | Forward primer 1 | Reverse primer 1 |
| | pET-22b-TfCut2-cSP | GAAGCTGTGGGTGGATAACGA CACCCGCTACACCCAGTTCCTC TG | CCGCGAAACGGTGTAGGTTGCC ACGGAGAAGGGGCCGCTG |
| | Template 2 | Forward primer 2 | Reverse primer 2 |
| | pET-22b-LCC-cSP | CAGCGGCCCCTTCTCCGTGGCA ACCTACACCGTTTCGC | AGATCGCCACCGCACCGAAGG TCAGCGAGGTGCCTGTGGG |
| | Template 3 | Forward primer 3 | Reverse primer 3 |
| | pET-22b-TfCut2-cSP | CACCTCGCTGACCTTCGGTGCG GTGGCGATCTCCCCCGGC | GCCGCCGCCCATCGAGTGGCCC ATGACCGCCAGTCGG |
| | Template 4 | Forward primer 4 | Reverse primer 4 |
| | pET-22b-LCC-cSP | GCGGTCATGGGCCACTCGATGG GCGGCGGCGGGAC | CGAGGTCGGCCCCGATGATCA GCACCGGTACCGACGTGTTGAA CG |
| | Template 5 | Forward primer 5 | Reverse primer 5 |
| | pET-22b-TfCut2-cSP | GTTCAACACGTCGGTACCGGTG CTGATCATCGGGGCCGACCTCG | GCTCCACGTACACCTTGCTGAT GGAGCTCGGCAGG |
| | Template 6 | Forward primer 6 | Reverse primer 6 |
| | pET-22b-LCC-cSP | CCGAGCTCCATCAGCAAGGTGT ACGTGGAGCTCGAC | CAGAGGAACTGGGTGTAGCGG GTGTCGTTATCCACCCACAGCT TC |
| 20 | Template 1 | Forward primer 1 | Reverse primer 1 |
| | pET-22b-TfCut2-cSP | GAAGCTGTGGGTGGATAACGA CACCCGCTACACCCAGTTCCTC TG | CCGCGAAACGGTGTAGGTTGCC ACGGAGAAGGGGCCGCTG |
| | Template 2 | Forward primer 2 | Reverse primer 2 |
| | pET-22b-LCC-cSP | CAGCGGCCCCTTCTCCGTGGCA ACCTACACCGTTTCGC | AGATCGCCACCGCACCGAAGG TCAGCGAGGTGCCTGTGGG |
| | Template 3 | Forward primer 3 | Reverse primer 3 |
| | pET-22b-TfCut2-cSP | CACCTCGCTGACCTTCGGTGCG GTGGCGATCTCCCCCGGC | GCTCCACGTACACCTTGCTGAT GGAGCTCGGCAGG |
| | Template 4 | Forward primer 4 | Reverse primer 4 |
| | pET-22b-LCC-cSP | CCGAGCTCCATCAGCAAGGTGT ACGTGGAGCTCGAC | CAGAGGAACTGGGTGTAGCGG GTGTCGTTATCCACCCACAGCT TC |

### Example 23.

The present example provides an engineered strain containing the recombinant plasmid of Example 22 and a construction method thereof. The construction method includes:

The recombinant plasmid of Example 22 was transformed into a BL21(DE3) competent cell and cultured using an ampicillin-containing medium to obtain a positive recombinant, which was the engineered strain containing the recombinant plasmid of Example 22.

### Example 24.

The present example provides a method for inducing expression the engineered strain of Example 23 and a method for purifying the target protein.

The engineered strain of Example 23 was inoculated into an LB liquid medium to culture overnight at 37°C, 220 r/min; the overnight culture solution was inoculated into a fresh LB liquid medium at an inoculation amount of 1% to culture at 37°C, 220 r/min to reach the OD600 about 0.8; IPTG with a volume percentage of 0.1% (v/v) was added to induce expression at 16°C for 20 h to obtain a fermentation culture. The fermentation culture was centrifuged at 4000 rpm for 15 min, and the centrifuged engineered strain wet cells were collected.

The centrifuged engineered strain wet cells were resuspended in a disrupting buffer, and the resuspended engineered strain wet cells were subjected to a disrupting treatment using a high-pressure cell disruption instrument. The disrupted solution was centrifuged at 10000 rpm for 60 min to remove cell debris, and the supernatant was loaded into a Ni-NTA column to absorb the target protein. The non-specifically adsorbed impurity protein was cleaned by a washing buffer. The target protein was eluted using an elution buffer and the eluate was concentrated via a protein concentration tube.

The formulations of LB liquid medium were: 5 g/L of yeast extract, 10 g/L of tryptone, 10 g/L of sodium chloride.

The formulations of disrupting buffer were: 50 mM Tris-HCl, 150 mM NaCl, 10 mM Imidazole, pH=7.5.

The formulations of washing buffer were: 50 mM Tris-HCl, 150 mM NaCl, 20 mM Imidazole, pH=7.5.

The formulations of elution buffer were: 50 mM Tris-HCl, 300 mM NaCl, 300 mM Imidazole, pH=7.5.

## Claims

1. An IsPETase-8×Chimera recombinase, **characterized by** comprising an amino acid sequence modified from an amino acid sequence of a wild-type polyethylene terephthalate, PET, hydrolase by replacing at least one of the amino acid sequences set forth in SEQ ID NOs: 1 to 8; wherein
the amino acid sequence set forth in SEQ ID NO: 1 is replaced by the amino acid sequence set forth in SEQ ID NO: 9 or SEQ ID NO: 10;
the amino acid sequence set forth in SEQ ID NO: 2 is replaced by the amino acid sequence set forth in SEQ ID NO: 11 or SEQ ID NO: 12;
the amino acid sequence set forth in SEQ ID NO: 3 is replaced by the amino acid sequence set forth in SEQ ID NO: 13 or SEQ ID NO: 14;
the amino acid sequence set forth in SEQ ID NO: 4 is replaced by the amino acid sequence set forth in SEQ ID NO: 15;
the amino acid sequence set forth in SEQ ID NO: 5 is replaced by the amino acid sequence set forth in SEQ ID NO: 16 or SEQ ID NO: 17;
the amino acid sequence set forth in SEQ ID NO: 6 is replaced by the amino acid sequence set forth in SEQ ID NO: 18;
the amino acid sequence set forth in SEQ ID NO: 7 is replaced by the amino acid sequence set forth in SEQ ID NO: 19; and
the amino acid sequence set forth in SEQ ID NO: 8 is replaced by the amino acid sequence set forth in SEQ ID NO: 20.

2. The IsPETase-8×Chimera recombinase of claim 1, wherein the amino acid sequences set forth in SEQ ID NOs: 2, 3, 4, 5 and 7 are replaced.

3. The IsPETase-8×Chimera recombinase of claim 1 or 2, wherein the amino acid sequence set forth in SEQ ID NO: 3 is replaced by the amino acid sequence set forth in SEQ ID NO: 14.

4. The IsPETase-8×Chimera recombinase of claim 1 or 2, wherein the amino acid sequence set forth in SEQ ID NO: 1 is replaced by the amino acid sequence set forth in SEQ ID NO: 9, the amino acid sequence set forth in SEQ ID NO: 2 is replaced by the amino acid sequence set forth in SEQ ID NO: 12, the amino acid sequence set forth in SEQ ID NO: 3 is replaced by the amino acid sequence set forth in SEQ ID NO: 14, the amino acid sequence set forth in SEQ ID NO: 4 is replaced by the amino acid sequence set forth in SEQ ID NO: 15, the amino acid sequence set forth in SEQ ID NO: 5 is replaced by the amino acid sequence set forth in SEQ ID NO: 17, the amino acid sequence set forth in SEQ ID NO: 6 is replaced by the amino acid sequence set forth in SEQ ID NO: 18, the amino acid sequence set forth in SEQ ID NO: 7 is replaced by the amino acid sequence set forth in SEQ ID NO: 19, and the amino acid sequence set forth in SEQ ID NO: 8 is replaced by the amino acid sequence set forth in SEQ ID NO: 20; or
the amino acid sequence set forth in SEQ ID NO: 1 is replaced by the amino acid sequence set forth in SEQ ID NO: 10, the amino acid sequence set forth in SEQ ID NO: 2 is replaced by the amino acid sequence set forth in SEQ ID NO: 12, the amino acid sequence set forth in SEQ ID NO: 3 is replaced by the amino acid sequence set forth in SEQ ID NO: 14, the amino acid sequence set forth in SEQ ID NO: 4 is replaced by the amino acid sequence set forth in SEQ ID NO: 15, the amino acid sequence set forth in SEQ ID NO: 5 is replaced by the amino acid sequence set forth in SEQ ID NO: 17, the amino acid sequence set forth in SEQ ID NO: 6 is replaced by the amino acid sequence set forth in SEQ ID NO: 18, the amino acid sequence set forth in SEQ ID NO: 7 is replaced by the amino acid sequence set forth in SEQ ID NO: 19, and the amino acid sequence set forth in SEQ ID NO: 8 is replaced by the amino acid sequence set forth in SEQ ID NO: 20; or
the amino acid sequence set forth in SEQ ID NO: 1 is replaced by the amino acid sequence set forth in SEQ ID NO: 9, the amino acid sequence set forth in SEQ ID NO: 2 is replaced by the amino acid sequence set forth in SEQ ID NO: 12, the amino acid sequence set forth in SEQ ID NO: 3 is replaced by the amino acid sequence set forth in SEQ ID NO: 14, the amino acid sequence set forth in SEQ ID NO: 4 is replaced by the amino acid sequence set forth in SEQ ID NO: 15, the amino acid sequence set forth in SEQ ID NO: 5 is replaced by the amino acid sequence set forth in SEQ ID NO: 16, the amino acid sequence set forth in SEQ ID NO: 7 is replaced by the amino acid sequence set forth in SEQ ID NO: 19, and the amino acid sequence set forth in SEQ ID NO: 8 is replaced by the amino acid sequence set forth in SEQ ID NO: 20; or
the amino acid sequence set forth in SEQ ID NO: 2 is replaced by the amino acid sequence set forth in SEQ ID NO: 12, the amino acid sequence set forth in SEQ ID NO: 3 is replaced by the amino acid sequence set forth in SEQ ID NO: 14, the amino acid sequence set forth in SEQ ID NO: 4 is replaced by the amino acid sequence set forth in SEQ ID NO: 15, the amino acid sequence set forth in SEQ ID NO: 5 is replaced by the amino acid sequence set forth in SEQ ID NO: 16, the amino acid sequence set forth in SEQ ID NO: 6 is replaced by the amino acid sequence set forth in SEQ ID NO: 18, the amino acid sequence set forth in SEQ ID NO: 7 is replaced by the amino acid sequence set forth in SEQ ID NO: 19, and the amino acid sequence set forth in SEQ ID NO: 8 is replaced by the amino acid sequence set forth in SEQ ID NO: 20; or
the amino acid sequence set forth in SEQ ID NO: 1 is replaced by the amino acid sequence set forth in SEQ ID NO: 9, the amino acid sequence set forth in SEQ ID NO: 2 is replaced by the amino acid sequence set forth in SEQ ID NO: 11, the amino acid sequence set forth in SEQ ID NO: 3 is replaced by the amino acid sequence set forth in SEQ ID NO: 14, the amino acid sequence set forth in SEQ ID NO: 4 is replaced by the amino acid sequence set forth in SEQ ID NO: 15, the amino acid sequence set forth in SEQ ID NO: 5 is replaced by the amino acid sequence set forth in SEQ ID NO: 16, the amino acid sequence set forth in SEQ ID NO: 6 is replaced by the amino acid sequence set forth in SEQ ID NO: 18, the amino acid sequence set forth in SEQ ID NO: 7 is replaced by the amino acid sequence set forth in SEQ ID NO: 19, and the amino acid sequence set forth in SEQ ID NO: 8 is replaced by the amino acid sequence set forth in SEQ ID NO: 20.

5. An application of the IsPETase-8×Chimera recombinase of any one of claims 1 to 4 for hydrolyzing PET, **characterized by** comprising: adding PET to a glycine-sodium hydroxide, NaOH, buffer with a pH of 8.0 to 10.0 and a concentration of 40 millimoles per liter, mM, to 60 mM so that a final concentration of PET is 60 milligrams per milliliter, mg/mL, to 100 mg/mL; adding the IsPETase-8×Chimera recombinase to the solution so that a final concentration of the IsPETase-8×Chimera recombinase is 400 nanomoles per liter, nM, to 600 nM; and incubating the mixture for 1 to 3 days at a temperature of 30°C to 50°C and a rotation speed of 100 revolutions per minute, rpm, to 300 rpm.

6. A nucleic acid, **characterized by** encoding the IsPETase-8×Chimera recombinase of any one of claims 1 to 4.

7. A recombinant plasmid, **characterized by** comprising the nucleic acid of claim 6.

8. A method for constructing the recombinant plasmid of claim 7, **characterized by** comprising:
using plasmid pET-22b-IsPETase, plasmid pET-22b-TfCut2 and plasmid pET-22b-LCC as templates, and performing PCR to obtain linear vector fragments corresponding to each template; mixing each of the linear vector fragments corresponding to each template with a ligase to form circular recombinant plasmids; transforming each of the circular recombinant plasmids into a T1 competent cell, and obtaining a recombinant plasmid pET-22b-IsPETase-cSP, a recombinant plasmid pET-22b-TfCut2-cSP and a recombinant plasmid pET-22b-LCC-cSP by ampicillin-containing medium screening and plasmid extraction; wherein a nucleotide sequence of an IsPETase-cSP gene in the recombinant plasmid pET-22b-IsPETase-cSP is set forth in SEQ ID NO: 51, and an amino acid sequence of an IsPETase-cSP enzyme is set forth in SEQ ID NO: 52; a nucleotide sequence of a TfCut2-cSP gene in the recombinant plasmid pET-22b-TfCut2-cSP is set forth in SEQ ID NO: 53, and an amino acid sequence of a TfCut2-cSP enzyme is set forth in SEQ ID NO: 54; a nucleotide sequence of a LCC-cSP gene in the recombinant plasmid pET-22b-LCC-cSP is set forth in SEQ ID NO: 55, and an amino acid sequence of a LCC-cSP enzyme is set forth in SEQ ID NO: 56;
using at least two of the recombinant plasmid pET-22b-IsPETase-cSP, the recombinant plasmid pET-22b-TfCut2-cSP and the recombinant plasmid pET-22b-LCC-cSP as templates to perform PCR separately to generate PCR amplified fragments, ligating the PCR amplified fragments to generate a ligation product, transforming the ligation product into a T1 competent cell, screening using the ampicillin-containing medium and performing plasmid extraction to obtain the recombinant plasmid.

9. An engineered strain, **characterized by** comprising the recombinant plasmid of claim 7.

10. A method for constructing the engineered strain of claim 9, **characterized by** comprising:
transforming the recombinant plasmid of claim 8 into a BL21(DE3) competent cell, and culturing the BL21(DE3) competent cell using an ampicillin-containing medium to obtain a positive recombinant.

11. An application of the engineered strain of claim 9 for hydrolyzing PET, **characterized by** comprising: culturing the engineered strain in an LB liquid medium to reach OD600 ranging from 0.7 to 0.9, adding 0.05% to 0.2% of IPTG to the medium, cooling to 14°C to 20°C to induce expression for 16 hours, h, to 24 h, centrifuging, and collecting wet cells of the engineered strain;
after breaking down the wet cells of the engineered strain, centrifuging to remove cell debris, eluting a centrifuged supernatant with an elution buffer as an elution solvent by using a Ni-NTA packing column, collecting an eluate, and concentrating the eluate to obtain a protein concentrate containing proteins;
adding the protein concentrate to a glycine-sodium hydroxide buffer with a pH of 8.0 to 10.0 and a concentration of 40 mM to 60 mM, so that the concentration of the proteins is 400 nM to 600 nM, and hydrolyzing PET at 30°C to 60°C; wherein a concentration of glycine in the glycine-sodium hydroxide buffer is 50 mM to 200 mM.
